# EUROPEAN PATENT SPECIFICATION

(11) **EP 2 550 916 B1**
(45) Date of publication and mention of the grant of the patent: **23.11.2016**
(21) Application number: 11759484.6
(22) Date of filing: 24.03.2011
(51) Int. Cl.: A61B 5/053, A61B 5/00

(54) **VISCERAL FAT MEASUREMENT DEVICE**
VORRICHTUNG ZUR VISZERALFETTMESSUNG
DISPOSITIF DE MESURE DES GRAISSES VISCÉRALES

(30) Priority: 24.03.2010 JP 2010068375
(43) Date of publication of application: 30.01.2013
(73) Proprietor: Panasonic Corporation, Kadoma-shi Osaka 571-8501 (JP)
(72) Inventor: TAKAHASHI, Tatsuya, Kadoma-shi Osaka 571-8686 (JP); FUKUDA, Hiroaki, Kadoma-shi Osaka 571-8686 (JP); FUKUSHIMA, Shogo, Kadoma-shi Osaka 571-8686 (JP); TERAZONO, Yoshie, Kadoma-shi Osaka 571-8686 (JP)
(74) Representative: Schwabe - Sandmair - Marx
(86) International application number: PCT/JP2011/057091
(87) International publication number: WO 2011/118679

(56) References cited:
- EP-A1- 1 935 338
- EP-A1- 1 958 567
- EP-A1- 1 989 999
- EP-A2- 1 712 179
- WO-A1-2007/043271
- DE-T5-112008 000 629

## Description

The present invention relates to a visceral fat measurement device.

A visceral fat measurement device measures visceral fat 82 included in a cross section defined by electrodes arranged around a measured body 70 illustrated in Fig. 5B (e.g., refer to Patent Document 1).

Patent Document 1: Japanese Laid-Open Patent Application No. 2002-369806

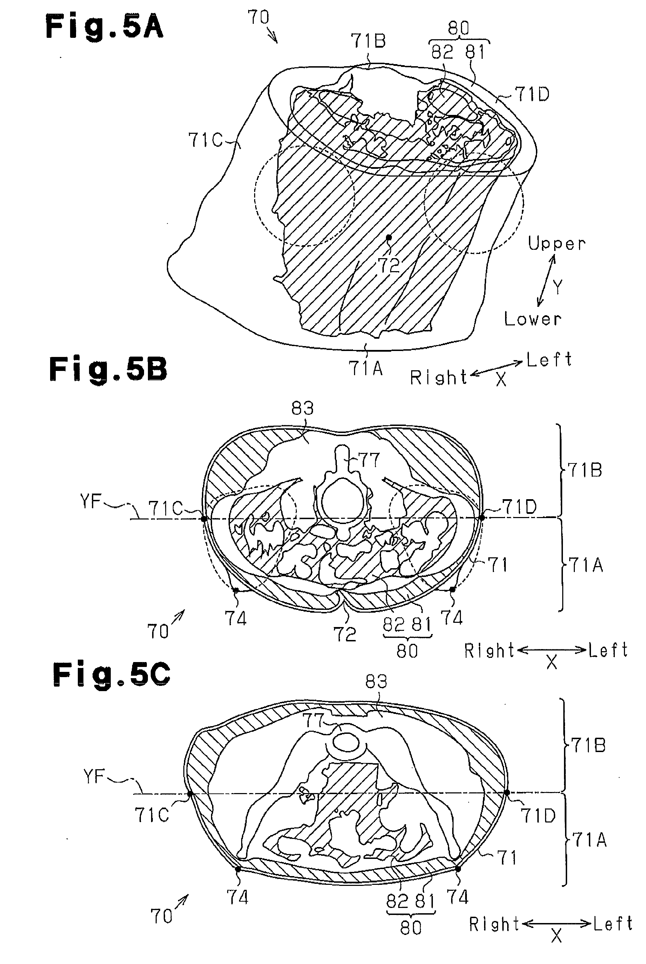

As illustrated in Fig. 5A, the visceral fat 82 is distributed three-dimensionally in fact. A conventional visceral fat measurement device provides a measurement result reflecting the visceral fat along the cross section of Fig. 5B, however, it is difficult to measure the volume of the visceral fat accurately.

EP 1 935 338 A1 describes a body fat measuring device which includes at least one pair of first electrodes to be disposed respectively at a first site and a second site sandwiching the abdomen of a subject, and an electrode group including a first abdomen electrode to be disposed at a surface of the abdomen of the subject. The first abdomen electrode includes a pair of second electrodes and a pair of third electrodes disposed in an alignment direction substantially perpendicular to a cross section of the abdomen. In a case where electric current is applied via the first electrodes, a first potential difference between electrodes of one predetermined pair included in the first abdomen electrode is detected. In a case where electric current is applied the third electrodes, a second potential difference between the second electrodes is detected. Based on the detected two types of potential differences and physical data of the subject, the visceral fat amount of the subject is calculated.

EP 1989 999 A1 relates to an apparatus for measuring body fat. In this respect, an apparatus has two current supply electrodes arranged on the circumferential surface of a subject with the distance between the electrodes being sufficiently shorter than a circumferential length of the subject; a first measuring electrode arranged on the subject in the vicinity of one of the current supply electrodes; a second measuring electrode arranged on the subject substantially opposite to the current supply electrodes across the subject or a second measuring electrode arranged on the subject in the vicinity of the other of the current supply electrodes; a voltage measuring unit to supply a current between the current supply electrodes and measure a voltage generated between the first and the second measuring electrodes; and a computing unit to compute a visceral fat quantity of the subject according to the measured voltage and a characteristic quantity representing the size of the subject.

EP 1 958 567 A1 describes a body fat measuring apparatus which includes: an upper limb unit including a hold portion that can be held in a hand of a subject and a main portion; and a lower limb electrode to be contacted with a lower limb of the subject. The hold portion includes an upper limb electrode, and the main portion includes a first surface to be contacted with a surface of an abdomen of the subject as well as a pair of first electrodes and a pair of second electrodes arranged at the first surface. The body fat measuring apparatus calculates a visceral fat amount of the subject based on two types of potential differences that are detected respectively in a case where an electric current is applied via a pair of the upper and lower limb electrodes and a case where an electric current is applied via the pair of second electrodes as well as physical data of the subject.

### SUMMARY OF THE INVENTION

The invention is defined by the subject-matter of the independent claims. The dependent claims are directed to advantageous embodiments.

### ADVANTAGES OF THE INVENTION

Advantageously, it is provided a visceral fat measurement device that can measure the volume of the visceral fat accurately.

Advantageously, a visceral fat measurement device includes a current-applying electrode pair including a first current-applying electrode and a second current-applying electrode, and a voltage-measuring electrode pair including a first voltage-measuring electrode and a second voltage-measuring electrode. The current-applying electrode pair is arranged on a projected line obtained by projecting a body trunk axis on a body surface in an abdominal part of a measured body. The first current-applying electrode and the second current-applying electrode are arranged apart from each other with an interval predetermined so that a current path connecting the first current-applying electrode, visceral fat, and the second current-applying electrode is formed. The voltage-measuring electrode pair is provided between the first current-applying electrode and the second current-applying electrode in a direction of the body trunk axis of the measured body.

It is preferable that the predetermined interval is larger than a total thickness of a subcutaneous fat layer and a muscle layer of a measured part.

It is preferable that the current-applying electrode pair is arranged in at least one region of a region between a line passing through an anterior superior iliac spine and a line passing through a coronal plane among the projected line obtained by projecting the body trunk axis on the body surface in a right half of the measured body, and a region between a line passing through an anterior superior iliac spine and a line passing through a coronal plane among the projected line obtained by projecting the body trunk axis on the body surface in a left half of the measured body.

It is preferable that the first current-applying electrode is arranged at an upper end of the abdominal part and the second current-applying electrode is arranged at a lower end of the abdominal part.

It is preferable that the first voltage-measuring electrode is arranged above a navel plane orthogonal to the body trunk axis at a height of a navel, and the second voltage-measuring electrode is arranged below the navel plane.

It is preferable that the first current-applying electrode, the first voltage-measuring electrode, the second voltage-measuring electrode, and the second current-applying electrode are arranged in line in this order.

It is preferable that the visceral fat measurement device includes a plurality of voltage-measuring electrode pairs and that the voltage-measuring electrode pair is each of, or one of the plurality of voltage-measuring electrode pairs.

It is preferable that the visceral fat measurement device includes a movable part for moving the voltage-measuring electrodes on the measured body.

It is preferable that the visceral fat measurement device includes a support member for bringing the visceral fat measurement device into contact with the measured body and that the current-applying electrode pair and the voltage-measuring electrode pair are supported by the support member.

It is preferable that the visceral fat measurement device includes a display unit for displaying a visceral fat amount based on a measured voltage of the voltage-measuring voltage pair and that the display unit is formed separately from the support member.

It is preferable that the support member includes a belt which is wound around the measured body and which fixes the current-applying electrode pair and the voltage-measuring electrode pair to the measured body.

It is preferable that the visceral fat measurement device includes a handle held by a user at measurement.

It is preferable that the support member includes disposing parts for detachably attaching the current-applying electrodes and the voltage-measuring electrodes.

It is preferable that the visceral fat measurement device includes a mark which indicates a reference position corresponding to a rib lower edge or an anterior superior iliac spine of the measured body to set positions of the current-applying electrode pair and the voltage-measuring electrode pair.

It is preferable that the visceral fat measurement device includes a computation circuit for calculating a volume of the visceral fat based on a measured voltage of the voltage-measuring electrode pair.

It is preferable that the visceral fat measurement device includes a display unit for displaying three-dimensional distribution of the visceral fat based on the measured voltage of the voltage-measuring electrode pair.

In one embodiment, the visceral fat measurement device includes a support member having a longitudinal axis. The first current-applying electrode and the second current-applying electrode are supported by the support member at an interval on a straight line which is orthogonal to, or substantially parallel to the longitudinal axis of the support member. The first voltage-measuring electrode and the second voltage-measuring electrode are supported by the support member between the first current-applying electrode and the second current-applying electrode.

A further aspect of the present invention provides a visceral fat measurement device that measures visceral fat by measuring voltage of a measured body when current is applied to the measured body. The visceral fat measurement device includes a current-applying electrode pair including a first current-applying electrode and a second current-applying electrode for applying current to the measured body, a voltage-measuring electrode pair including a first voltage-measuring electrode and a second voltage-measuring electrode for measuring voltage of the measured body, and a support member having a longitudinal axis. The first current-applying electrode and the second current-applying electrode are supported by the support member at an interval on a straight line which is orthogonal to, or substantially parallel to the longitudinal axis of the support member. The voltage-measuring electrode pair is supported by the support member between the first current-applying electrode and the second current-applying electrode.

In one embodiment, the first current-applying electrode, the second current-applying electrode, the first voltage-measuring electrode, and the second voltage-measuring electrode are aligned on a common straight line orthogonal to the longitudinal axis of the support member.

In one embodiment, the first current-applying electrode and the second current-applying electrode are arranged on a first straight line orthogonal to the longitudinal axis of the support member. The first voltage-measuring electrode and the second voltage-measuring electrode are arranged on a second straight line that is parallel to the first straight line.

In one embodiment, the support member is configured to fix the visceral fat measurement device to the measured body. The current-applying electrode pair is supported by the support member at a position determined so that the first current-applying electrode and the second current-applying electrode are arranged automatically on a projected line obtained by projecting a body trunk axis of the measured body on a body surface of an abdominal part of the measured body when the visceral fat measurement device is fixed to the measured body by the support member.

In one embodiment, the support member is a flexible support member that can be wound around the measured body.

In one embodiment, the interval is determined so that a current path connecting the first current-applying electrode, the visceral fat, and the second current-applying electrode is formed.

The present invention provides a visceral fat measurement device that can measure the volume of the visceral fat accurately.
Fig. 1 is a block diagram of a visceral fat measurement device according to a first embodiment of the present invention.
Figs. 2A and 2B are a rear view and a front view of the visceral fat measurement device of Fig. 1, respectively.
Fig. 3 is a schematic view of the visceral fat measurement device before attachment to a measured body.
Fig. 4 is a schematic view of the visceral fat measurement device attached to a measured body.
Fig. 5A is a partial cutaway perspective view of a measured body.
Fig. 5B is a sectional view of the measured body at the height of a navel.
Fig. 5C is a sectional view of the measured body at the height of an ilium.
Fig. 6 is a schematic view indicating the positions of electrodes in the visceral fat measurement device attached to a measured body.
Fig. 7 is a schematic sectional view of the visceral fat measurement device of Fig. 6.
Fig. 8 is a schematic view indicating the positions of electrodes in a visceral fat measurement device according to a second embodiment of the present invention.
Fig. 9 is a schematic view indicating the positions of electrodes in a visceral fat measurement device according to a third embodiment of the present invention.
Fig. 10 is a schematic view indicating the positions of electrodes in a visceral fat measurement device according to a fourth embodiment of the present invention.
Fig. 11 is a schematic view indicating the positions of electrodes in a visceral fat measurement device according to a fifth embodiment of the present invention.
Fig. 12 is a schematic view of a visceral fat measurement device according to a sixth embodiment of the present invention.
Fig. 13 is a schematic view of a visceral fat measurement device according to a seventh embodiment of the present invention.
Fig. 14 is a rear view of a visceral fat measurement device according to an eighth embodiment of the present invention.
Fig. 15 is a schematic view indicating the positions of electrodes in a first modified example.
Fig. 16A is a schematic view illustrating a visceral fat measurement device in a second modified example.
Fig. 16B is a schematic view illustrating a visceral fat measurement device in a third modified example.
Fig. 17 is a schematic view of a visceral fat measurement device in a fourth modified example.

### First embodiment

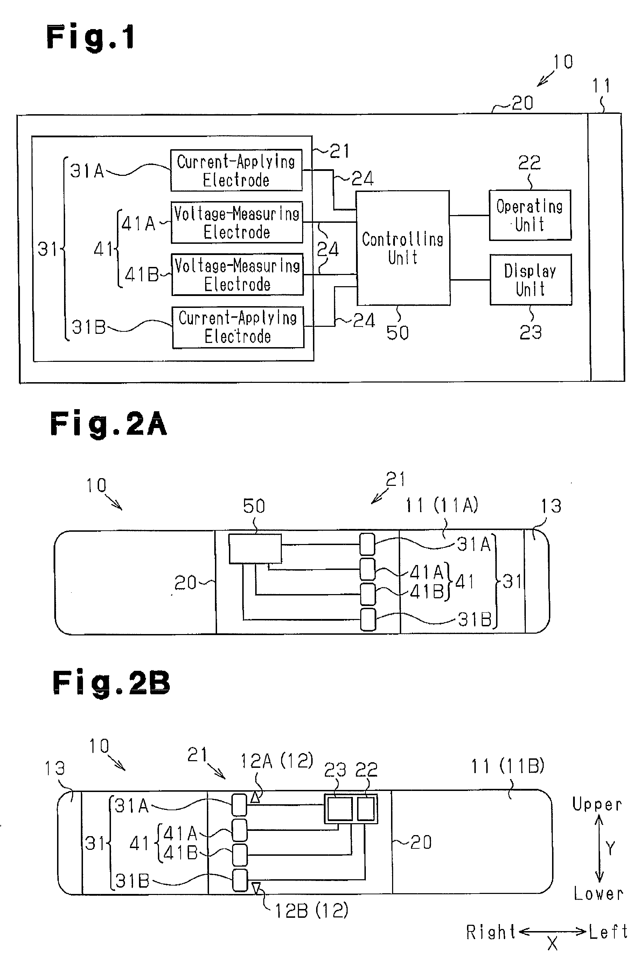

A first embodiment of the present invention is described with reference to Figs. 1 to 7.

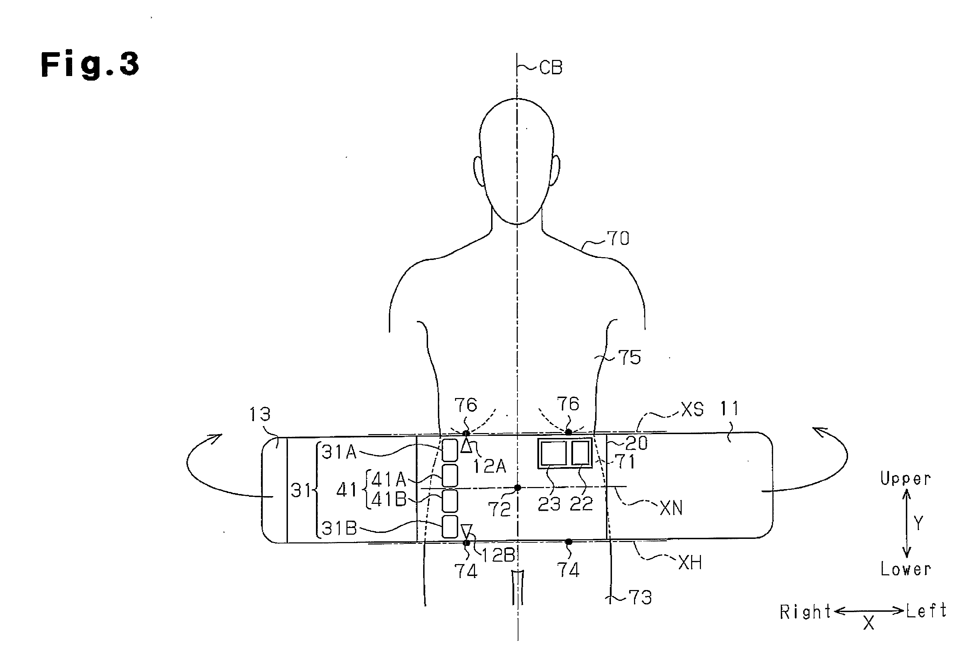

As illustrated in Fig. 1, a visceral fat measurement device 10 includes a measuring part 20 for measuring visceral fat, and a belt 11 for winding the measurement device 10 around the measured body 70 and fixing the measurement device 10 to the measured body 70. The measured body 70 may be a human body illustrated in Fig. 3.

The measuring part 20 includes a detection plane 21 including a plurality of electrodes for detecting voltage by applying current to the measured body 70, an operating unit 22 like an input panel, a display unit 23 for indicating various information to a measurer, and a controlling unit 50 connected to the electrodes of the detection plane 21.

The plurality of electrodes of the detection plane 21 include a current-applying electrode pair 31 for applying current to the measured body 70, and a voltage-measuring electrode pair 41 for measuring the voltage of the measured body 70. The current-applying electrode pair 31 includes two current-applying electrodes 31A and 31B. The voltage-measuring electrode pair 41 includes two voltage-measuring electrodes 41 A and 41 B. The current-applying electrodes 31 A and 31B and the voltage-measuring electrodes 41 A and 41B are connected to the controlling unit 50 through transmission lines 24. The current-applying electrodes 31A and 31B apply current to the measured body 70 in accordance with a control signal or a drive signal supplied from the controlling unit 50, and the voltage-measuring electrodes 41 A and 41B provide measurement signals corresponding to the measured voltage of the measured body 70 to the controlling unit 50.

The operating unit 22 can provide information on the measured body 70, such as the constitution of the measured body 70, to the controlling unit 50 in accordance with the operation of a measurer, for example. The operating unit 22 can provide a signal for starting the measurement of visceral fat to the controlling unit 50 in response to the operation of a measurer. In this case, the controlling unit 50 starts the application of current by controlling the current-applying electrode pair 31. The voltage-measuring electrode pair 41 measures the voltage value of the measured body 70, and transmits the measurement signal corresponding to the measured voltage value to the controlling unit 50. The controlling unit 50 calculates the visceral fat amount, especially the volume of visceral fat, based on the measured voltage value (measurement signal) and the information on the measured body and the like that are input via the operating unit 22, and displays the calculated visceral fat amount on the display unit 23. In an example, the controlling unit 50 creates a three-dimensional image representing the distribution of visceral fat based on the measured voltage value, the information on the measured body such as abdominal circumference, age, and sex input via the operating unit 22, other parameters, and computer algorithm, and displays the image on the display unit 23. The controlling unit 50 functions as a computation circuit.

With reference to Figs. 2A and 2B, the structure of the visceral fat measurement device 10 is described. The visceral fat measurement device 10 includes a rear surface 11A illustrated in Fig. 2A and a front surface 11B illustrated in Fig. 2B. The rear surface 11A faces the measured body 70 when the visceral fat measurement device 10 is wound around the measured body 70. The rear surface 11A is provided with the detection plane 21. The front surface 11B is opposite to the rear surface 11A. In the description below, the longitudinal direction of the visceral fat measurement device 10 may be referred to as "horizontal direction X". In a state that the visceral fat measurement device 10 is wound around the measured body 70, parts of the visceral fat measurement device 10 corresponding to the right half and the left half of the measured body 70 may be referred to as a right side and a left side, respectively. A direction orthogonal to the horizontal direction X may be referred to as "vertical direction Y". In the state that the visceral fat measurement device 10 is wound around the measured body 70, a side thereof close to the top of the head of the measured body 70 may be referred to as an upper side.

As illustrated in Figs. 2A and 2B, the visceral fat measurement device 10 includes a support member for supporting the electrodes. The support member may be the belt 11 wound around the measured body 70 in close contact. The belt 11 has both ends at locations extended in the horizontal direction X from the measuring part 20. The measuring part 20 may be a part of the belt 11. The belt 11 is an example of a flexible rectangular support member that can be wound around the measured body.

An electrode surface of each electrode of the current-applying electrode pair 31 and the voltage-measuring electrode pair 41 is exposed to the detection plane 21. The electrode material may be a stainless steel or a resin material plated with metal. The measuring part 20 can incorporate a power supply used for the visceral fat measurement device 10. As illustrated in Fig. 2B, the front surface 11B of the visceral fat measurement device 10 is provided with a mark 12, the operating unit 22, and the display unit 23.

The mark 12 serves as a positioning mark used for attaching the measuring part 20 including the electrodes to the measured body 70, and may be two marks 12A and 12B, for example. In the illustrated example, one mark 12A is located at an upper end and on the right side from a center of the measuring part 20, while the other mark 12B is located on the right side from the center and at a lower end of the measuring part 20. The locations of the electrodes on the visceral fat measurement device 10 may be determined based on the mark 12.

Fasteners 13 are provided at a right end part of the rear surface 11A and a left end part of the front surface 11B. By attaching the fasters 13 of the front surface 11B and the rear surface 11A to each other after the belt 11 is wound around the measured body 70, the visceral fat measurement device 10 is fixed to the measured body 70.

The current-applying electrodes 31A and 31B and the voltage-measuring electrodes 41A and 41B are located at equal intervals on a straight line extending in the vertical direction Y in the order of the electrode 31A, the electrode 41A, the electrode 41B, and the electrode 31B, in the right end part of the measuring part 20.

The more specific positions of the electrodes seen from the front surface 11B are briefly described below:
The electrode 31A, the electrode 41A, the electrode 41B, and the electrode 31B are located at the same position in the horizontal direction X;
The electrode 31A is located at the upper end part of the measuring part 20 in the vertical direction Y;
The electrode 31B is located at the lower end part of the measuring part 20 in the vertical direction Y;
The electrode 41A is located closer to the electrode 31A between the electrode 31A and the electrode 31B in the vertical direction Y; and
The electrode 41B is located below the electrode 41A between the electrode 31A and the electrode 31B in the vertical direction Y.

In this manner, the current-applying electrodes 31A and 31B and the voltage-measuring electrodes 41A and 41B are located at the same position in the horizontal direction X. The voltage-measuring electrodes 41A and 41B are located between the current-applying electrodes 31 A and 31B in the vertical direction Y.

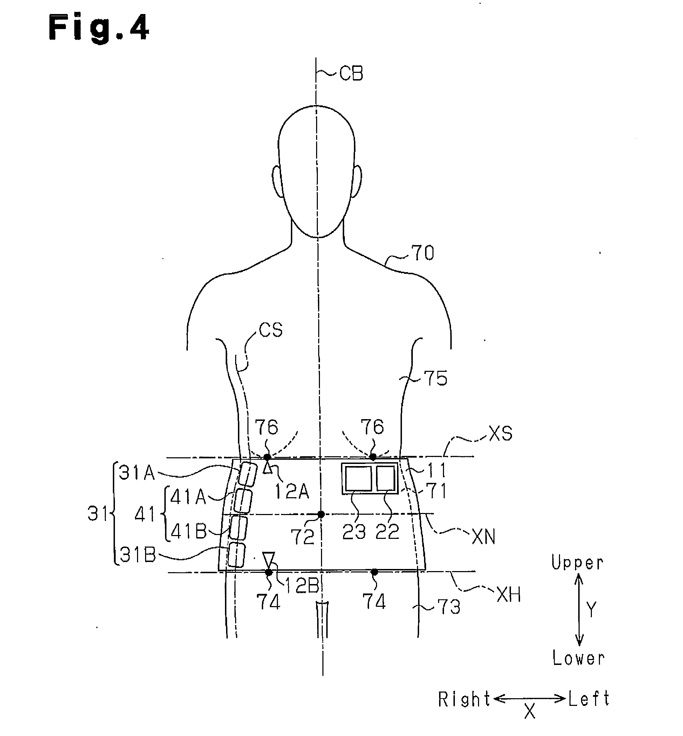

With reference to Fig. 3 and Fig. 4, the procedure of attaching the visceral fat measurement device 10 to the measured body 70 is described.

Here, the longitudinal axis of a body trunk of the measured body 70 is defined as "body trunk axis CB". A plane passing through a rib lower edge 76 and intersecting with the body trunk axis CB perpendicularly is defined as "rib plane XS". A plane passing through an anterior superior iliac spine 74 (see Fig. 5) and being orthogonal to the body trunk axis CB is defined as "ilium plane XH". A plane passing through a navel 72 and being orthogonal to the body trunk axis CB is defined as "navel plane XN". The right half and the left half of the body correspond to the parts more on the right and left sides than the navel 72, respectively. The part ranging from the rib plane XS to the ilium plane XH is defined as an abdominal part 71, the part above the rib plane XS is defined as a breast part 75, and the part below the ilium plane XH is defined as a hip part 73.

As illustrated in Fig. 3, the rear surface 11A of the belt 11 is attached to the abdominal part 71 of the measured body 70 (see Fig. 2A), and the position of the mark 12A in the horizontal direction X is aligned at the rib lower edge 76. The position of the mark 12A in the vertical direction Y is aligned at the vicinity of the rib plane XS and below the plane XS. The position of the mark 12B in the horizontal direction X is aligned at the anterior superior iliac spine 74. The position of the mark 12B in the vertical direction Y is aligned at the vicinity of the ilium plane XH and above the plane XH. The belt 11 is wound around the measured body 70 so that the visceral fat measurement device 10 is fixed to the measured body 70 with the fastener 13.

As illustrated in Fig. 4, when the belt 11 is wound around the abdominal part 71 of the measured body 70, the current-applying electrode pair 31 and the voltage-measuring electrode pair 41 are in contact with a body surface of the measured body 70. In this manner, by winding the belt 11 around the measured body 70, the electrodes 31A, 31B, 41A, and 41B are arranged automatically on a projected line CS obtained by projecting the body trunk axis CB on the body surface. When a measurer operates the operating unit 22 in this state, the measurement of the visceral fat 82 is started.

With reference to Fig. 5, body fat 80 of the measured body 70 is described. Here, a plane passing through a boundary between a front part 71A and a rear part 71B of the measured body 70 and being in parallel to the body trunk axis CB is defined as "coronal plane YF". A part of the right half of the body, which coincides with the coronal plane YF, is defined as a right flank 71C. A part of the left half of the body, which coincides with the coronal plane YF, is defined as a left flank 71D.

Fig. 5A illustrates the three-dimensional distribution of the body fat 80 ranging from the rib plane XS to the ilium plane XH of the measured body 70. Fig. 5B illustrates the distribution of the body fat 80 on the navel plane XN of the measured body 70. Fig. 5C illustrates the distribution of the body fat 80 on the ilium plane XH of the measured body 70. Note that the hatching pattern of Fig. 5A represents the distribution of the body fat 80. The hatching patterns of Figs. 5B and 5C each represent the distribution of the body fat 80 including subcutaneous fat 81 and the visceral fat 82.

As illustrated in Fig. 5A and Fig. 5B, the subcutaneous fat 81 exists in a layer form on the periphery below the body surface of the measured body 70 along the cross section passing through the navel 72 of the measured body 70 and being perpendicular to the body trunk axis CB. The layer of the subcutaneous fat 81 is thick in the front part 71A and the rear part 71B, and is relatively thin in the left flank 71D and the right flank 71C. The visceral fat 82 exists between the internal organs in the abdominal cavity. In general, the body fat 80 is distributed approximately symmetrically in the right half and left half of the body with respect to a backbone 77. The volume of the body fat 80 of the measured body 70 is different depending on the height in the measured body 70, i.e., different for every cross-sectional position orthogonal to the body trunk axis CB.

As illustrated in Figs. 5B and 5C, when the ilium plane XH is seen, the anterior superior iliac spine 74 exists displaced from each of the right flank 71C and the left flank 71D toward the navel 72. The visceral fat 82 is particularly distributed between the right flank 71C in the front part 71A and the anterior superior iliac spine 74 of the right half of the body and between the left flank 71D and the anterior superior iliac spine 74 of the left half of the body in the upper side of the abdominal part 71. Moreover, the distribution of the visceral fat 82 is biased on the outer periphery side rather than in the center in the abdominal cavity surrounded by a muscle 83.

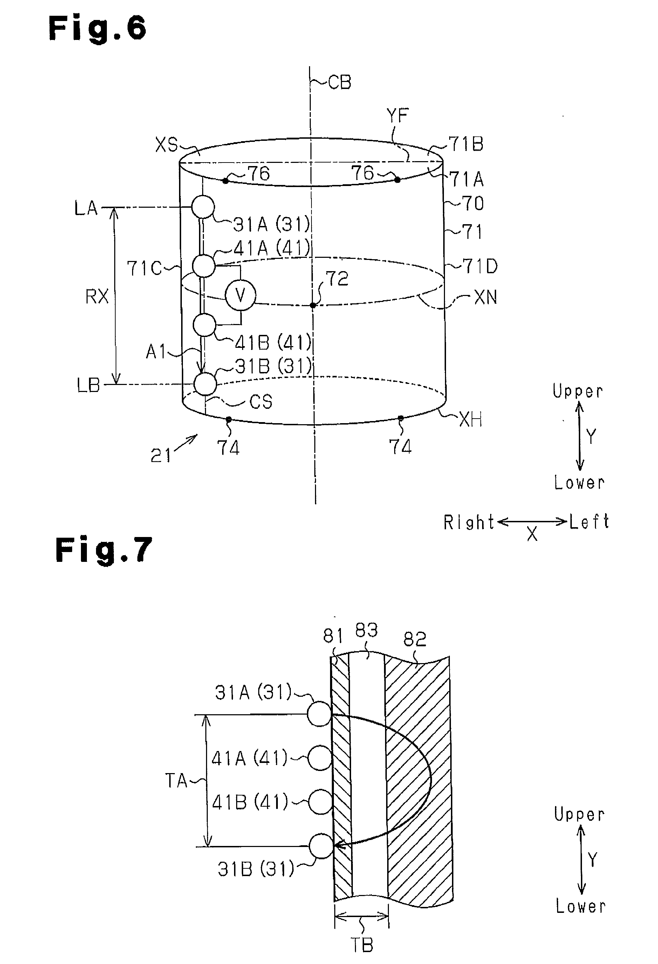

With reference to Fig. 6 and Fig. 7, the arrangement of the current-applying electrode pair 31 and the voltage-measuring electrode pair 41 for the measured body 70 and a method for measuring the body fat 80 are described. A cylindrical shape of Fig. 6 represents the abdominal part 71 of Fig. 4 around which the visceral fat measurement device 10 is wound.

Hereinafter, a line connecting the current-applying electrode 31A with the current-applying electrode 31B is defined as "inter-electrode line A1". The position of the current-applying electrode 31 A in the vertical direction Y is defined as a vertical position LA. The position of the current-applying electrode 31B in the vertical direction Y is defined as a vertical position LB.

As illustrated in Fig. 6, the current-applying electrode pair 31 is provided particularly between the right flank 71C and the anterior superior iliac spine 74 in the right half of the body. In an example, the current-applying electrode 31A is provided between the right flank 71C and the anterior superior iliac spine 74 and below the rib plane XS. The current-applying electrode 31B is provided between the right flank 71C and the anterior superior iliac spine 74 and above the ilium plane XH.

When the range from the vertical position LA to the vertical position LB in the vertical direction Y is defined as "inter-electrode range RX", the arrangement of the voltage-measuring electrode pair 41 can be explained as below.

The voltage-measuring electrode pair 41 (electrodes 41A and 41B) is arranged between the vertical position LA and the vertical position LB. The electrodes 41A and 41B are arranged on the inter-electrode line A1 and on the projected line CS. The intermediate position of the voltage-measuring electrode pair 41 coincides with the intermediate position of the current-applying electrode pair 31. In the illustrated example, their intermediate positions coincide with the navel plane XN.

As illustrated in Fig. 7, a distance TA between the current-applying electrode 31A and the current-applying electrode 31B is determined so as to be larger than layer thickness TB of the subcutaneous fat 81 and the muscle 83 (generally, 10 to 40 mm). Therefore, the voltage measured by the voltage-measuring electrode pair 41 when the current-applying electrode pair 31 applies current to the measured body 70 is the one reflecting the volume of the visceral fat 82 well.

Next, the measurement of the visceral fat 82 is explained.

The current fed from the current-applying electrode 31A to the current-applying electrode 31B flows inside the measured body 70 along the body trunk axis CB. When the current flows inside the measured body 70, the voltage changes depending on the composition of the measured body 70. The change in voltage is different depending on the resistance values of the subcutaneous fat 81, the visceral fat 82, and the muscle 83.

When the measurement of the visceral fat 82 is started, the current flows between the current-applying electrodes 31A and 31B. The voltage value when the current flows inside the measured body 70 is measured by the voltage-measuring electrodes 41A and 41B. Based on the measured voltage value, the controlling unit 50 calculates the amount of the visceral fat 82 between the electrodes 31A and 31B and in the vicinity of these electrodes 31A and 31B, i.e., the volume of the visceral fat 82 in the right half of the body, and displays the calculated result on the display unit 23. Since various known methods can be employed as a calculation method for the volume of the visceral fat, the description is omitted.

As described above, the present embodiment can provide the following effects.
(1) In the present embodiment, the current-applying electrode pair 31 is provided on the projected line CS obtained by projecting the body trunk axis CB on the body surface in the abdominal part 71 of the measured body 70. The distance TA between the current-applying electrode 31A and the current-applying electrode 31B is larger than the layer thickness TB of the subcutaneous fat 81 and the muscle 83 of the measured body 70. The voltage-measuring electrode pair 41 measures the voltage when the current-applying electrode pair 31 applies current and the current flows through the measured body 70 in the vertical direction Y. Therefore, the measurement results based on the stereoscopic distribution of the visceral fat 82 can be obtained.

The distance TA between the current-applying electrodes 31A and the current-applying electrodes 31B is larger than the thickness TB including the subcutaneous fat layer and the muscle layer at the measuring part of the measured body 70. That is, the distance TA is determined so that a current path connecting the electrode 31A, the visceral fat 82, and the electrode 31B is formed. Therefore, the voltage reflecting the volume of the visceral fat 82 can be detected by the voltage-measuring electrode pair 41. Thus, the volume of the visceral fat 82 can be measured accurately.

With the electrode arrangement in which the voltage-measuring electrode pair 41 is provided between the current-applying electrodes 31A and the current-applying electrode 31B in a direction substantially along the body trunk axis CB of the measured body 70, the voltage can be detected with high sensitivity as compared with the electrode arrangement in which the voltage-measuring electrode pair 41 is provided outside between the electrode 31A and the electrode 31B.
(2) In the abdominal part 71, the visceral fat 82 is most likely to appear between the anterior superior iliac spine 74 and the right flank 71C in the right half of the body and between the anterior superior iliac spine 74 and the left flank 71D in the left half of the body. In the present embodiment, since the current-applying electrode pair 31 is arranged between the anterior superior iliac spine 74 and the right flank 71C in the right half of the body, the voltage well reflecting the part containing the visceral fat 82 the most in the right half of the body can be detected. Since the part containing the visceral fat 82 much and the amount of the entire visceral fat 82 are closely correlated with each other, the volume of the visceral fat 82 can be measured accurately.
(3) In the present embodiment, the current-applying electrode 31A is arranged in the vicinity of the rib lower edge 76 and the current-applying electrode 31B is arranged in the vicinity of the anterior superior iliac spine 74. Therefore, the current of the current-applying electrode pair 31 reaches the center of the abdominal part 71, which makes it possible to detect the voltage well reflecting the total amount of the visceral fat 82 in the right half of the body.
(4) In the direction along the body trunk axis CB in the abdominal part 71, the visceral fat 82 is most likely to appear in the vicinity of the navel plane XN. In the present embodiment, the voltage-measuring electrode 41A is arranged above the navel plane XN, and the voltage-measuring electrode 41B is arranged below the navel plane XN. Therefore, the voltage well reflecting the part containing the visceral fat 82 the most can be detected. Moreover, the amount of body fat at the navel plane XN is used as a reference value for determination of the obesity or the metabolic syndrome. Therefore, by calculating the value well reflecting the visceral fat 82 in the vicinity of the navel plane XN in this manner, the comparison with the above reference value can be performed.
(5) In the present embodiment, the current-applying electrode 31A, the voltage-measuring electrode 41A, the voltage-measuring electrode 41B, and the current-applying electrode 31B are arranged in this order in line. Therefore, the distance between the current-applying electrode pair 31 and the voltage-measuring electrode pair 41 is smaller than that in the case where the electrodes 31A, 41A, 41B, and 31B are not arranged in line. This allows the voltage to be measured with high sensitivity.
(6) In the present embodiment, the belt 11 is provided with the current-applying electrode pair 31 and the voltage-measuring electrode pair 41. Therefore, the positional relation between the electrodes of the current-applying electrode pair 31 and the voltage-measuring electrode pair 41 does not change for every measurement of the visceral fat 82, which can reduce the variation in measurement result of the visceral fat 82.
(7) The visceral fat measurement device 10 includes the belt 11 for fixing the measuring part 20 to the measured body 70. Therefore, the displacement of the electrode pairs 31 and 41 from the measured body 70 can be reduced as compared with the case where the visceral fat 82 is measured while a measurer holds the measuring part 20 with a hand. Moreover, since the measurer does not need to hold the measuring part 20 at the measurement of the visceral fat 82, the burden on the measurer can be reduced.
(8) The visceral fat measurement device 10 includes the mark 12B indicating the reference position corresponding to the anterior superior iliac spine 74 of the measured body 70 and the mark 12A indicating the reference position corresponding to the rib lower edge 76 of the measured body 70. The positions of the current-applying electrode pair 31 and the voltage-measuring electrode pair 41 are set based on the marks 12A and 12B. Therefore, it is possible to suppress the large difference in position of the current-applying electrode pair 31 and the voltage-measuring electrode pair 41 for each measurement of the visceral fat 82, which allows the appropriate measurement on change over time of the visceral fat 82 on the same part of the measured body 70.
(9) The controlling unit 50 calculates the volume of the visceral fat 82 based on the voltage measured by the voltage-measuring electrode pair 41. Therefore, a measurer can know the volume of the visceral fat 82 of each part of the measured body 70.
(10) The visceral fat measurement device 10 includes the display unit 23 for displaying the three-dimensional distribution of the visceral fat 82. Therefore, a measurer can visually recognize the stereoscopic distribution of the visceral fat 82.

### Second embodiment

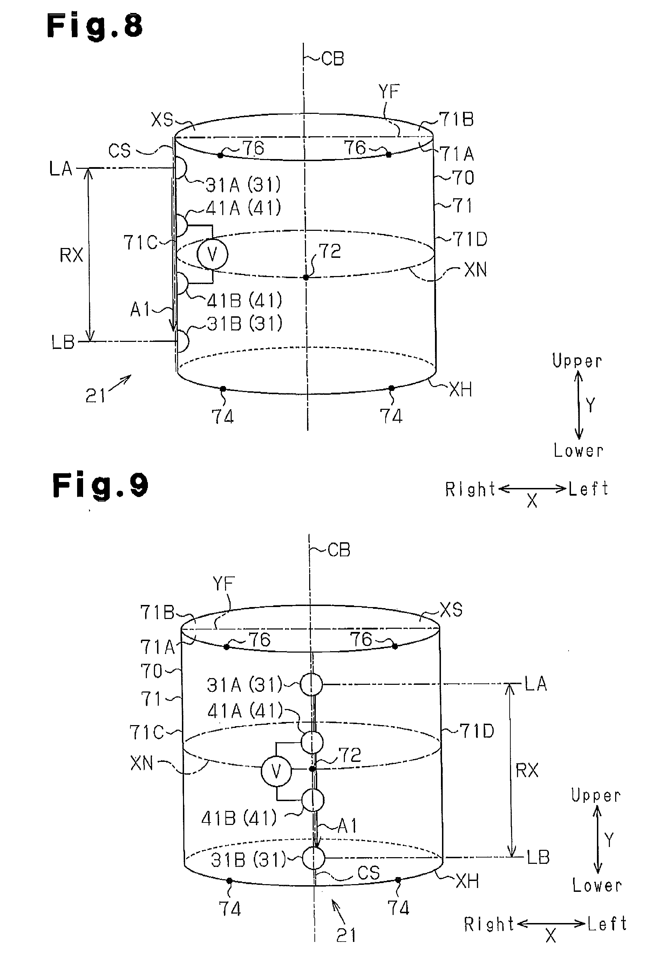

With reference to Fig. 8, a point of the second embodiment of the present invention that is different from the first embodiment is described. This embodiment is different from the first embodiment in the arrangement of the current-applying electrodes the voltage-measuring electrodes. The other points are similar to those in the first embodiment; therefore, the similar structures are denoted by the same reference symbols and description thereof is omitted.

As illustrated in Fig. 8, the current-applying electrode pair 31 and the voltage-measuring electrode pair 41 on the detection plane 21 are arranged in line on the projected line CS in the right flank 71C in the order of the electrodes 31A, 41A, 41B, and 31B.

The current-applying electrode 31A is arranged in the vicinity of the right flank 71C and below the rib plane XS. The current-applying electrode 31B is arranged in the vicinity of the right flank 71C and above the ilium plane XH.

The voltage-measuring electrode pair 41 (electrodes 41A and 41B) is arranged between the vertical position LA and the vertical position LB, i.e., in the inter-electrode range RX. The electrodes 41A and 41B are arranged on the inter-electrode line A1 and on the projected line CS. The intermediate position of the voltage-measuring electrode pair 41 coincides with the intermediate position of the current-applying electrode pair 31. In the illustrated example, their intermediate positions coincide with the navel plane XN.

As described above, the present embodiment can provide the effects similar to (1) and (3) to (10).

### Third embodiment

With reference to Fig. 9, a point of the third embodiment of the present invention that is different from the first embodiment is described. This embodiment is different from the first embodiment in the arrangement of the current-applying electrodes and the voltage-measuring electrodes. The other points are similar to those in the first embodiment; therefore, the similar structures are denoted by the same reference symbols and description thereof is omitted.

As illustrated in Fig. 9, the current-applying electrode pair 31 and the voltage-measuring electrode pair 41 on the detection plane 21 are arranged so that the electrodes 31A, 41A, 41B, and 31B are arranged in this order in line on the projected line CS passing through the navel 72.

The current-applying electrode 31A is arranged below the rib plane XS. The current-applying electrode 31B is arranged above the ilium plane XH.

As for the voltage-measuring electrode pair 41 (electrodes 41A and 41B), the electrodes 41A and 41B are arranged on the inter-electrode line A1 between the vertical position LA and the vertical position LB, that is, in the inter-electrode range RX. The intermediate position of the voltage-measuring electrode pair 41 coincides with the intermediate position of the current-applying electrode pair 31. In the illustrated example, their intermediate positions coincide with the navel plane XN.

As thus described above, the present embodiment can provide the effects similar to (1) and (3) to (10).

### Fourth embodiment

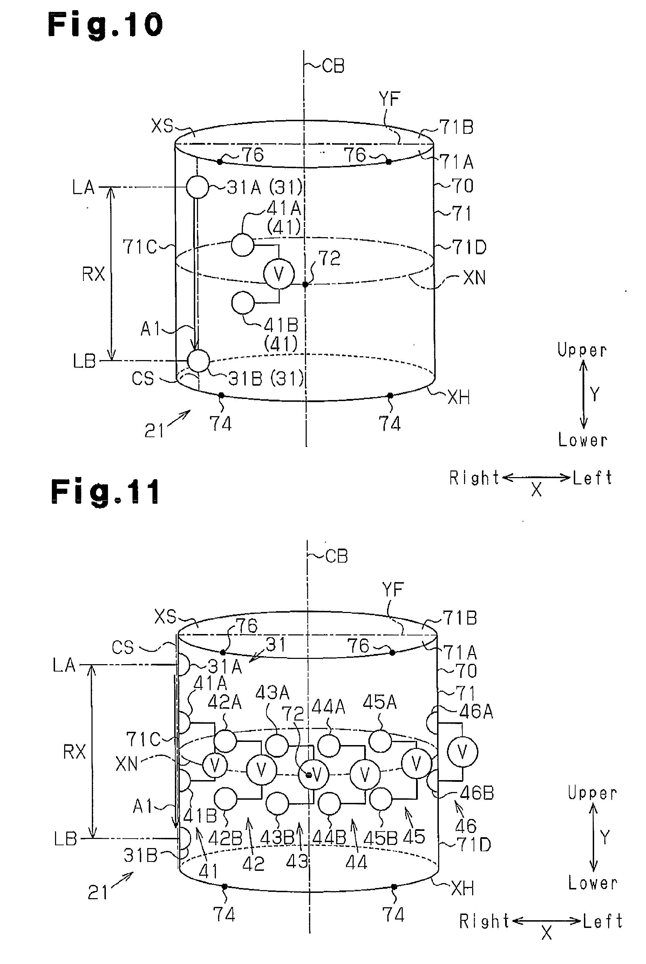

With reference to Fig. 10, a point of the fourth embodiment of the present invention that is different from the first embodiment is described. This embodiment is different from the first embodiment in the arrangement of the voltage-measuring electrodes. The other points are similar to those in the first embodiment; therefore, the similar structures are denoted by the same reference symbols and description thereof is omitted.

As illustrated in Fig. 10, the current-applying electrode pair 31 on the detection plane 21 is provided between the anterior superior iliac spine 74 and the right flank 71C in the right half of the body. In an example, the current-applying electrode 31A is arranged between the right flank 71C and the anterior superior iliac spine 74 and below the rib plane XS. The current-applying electrode 31B is arranged between the right flank 71C and the anterior superior iliac spine 74 and above the ilium plane XH.

The voltage-measuring electrode pair 41 (electrodes 41A and 41B) is arranged between the vertical position LA and the vertical position LB, that is, in the inter-electrode range RX. The electrodes 41A and 41B are arranged at positions away from the inter-electrode line A1 toward the navel 72. The intermediate position of each of the voltage-measuring electrode pair 41 and the current-applying electrode pair 31 coincides with the navel plane XN.

As described above, the present embodiment can provide the effects similar to (1) to (4) and (6) to (10).

### Fifth embodiment

With reference to Fig. 11, a point of the fifth embodiment of the present invention that is different from the second embodiment is described. This embodiment is different from the second embodiment in the arrangement of the voltage-measuring electrodes. The other points are similar to those in the second embodiment; therefore, the similar structures are denoted by the same reference symbols and description thereof is omitted.

As illustrated in Fig. 11, the current-applying electrode pair 31 and six voltage-measuring electrode pairs 41, 42, 43, 44, 45, and 46 are arranged on the detection plane 21. The voltage-measuring electrode pair 42 includes an electrode 42A and an electrode 42B. The voltage-measuring electrode pair 43 includes an electrode 43A and an electrode 43B. The voltage-measuring electrode pair 44 includes an electrode 44A and an electrode 44B. The voltage-measuring electrode pair 45 includes an electrode 45A and an electrode 45B. The voltage-measuring electrode pair 46 includes an electrode 46A and an electrode 46B.

The electrodes 31A, 41A, 41B, and 31B forming the current-applying electrode pair 31 and the voltage-measuring electrode pair 41 are arranged in line in this order on the projected line CS.

The electrode pair 42 is arranged in parallel to the electrode pair 41 in the horizontal direction X and at positions away from the electrode pair 41 toward the navel 72 in the right half of the body.

The electrode pair 43 is arranged in parallel to the electrode pair 42 in the horizontal direction X and at positions away from the electrode pair 42 toward the navel 72 in the right half of the body.

The electrode pair 44 is arranged in parallel to the electrode pair 43 in the horizontal direction X and in the vicinity of the navel 72 in the left half of the body.

The electrode pair 45 is arranged in parallel to the electrode pair 44 in the horizontal direction X and at positions away from the electrode pair 44 toward the left flank 71D in the left half of the body.

The electrode pair 46 is arranged in parallel to the electrode pair 45 in the horizontal direction X and on the left flank 71D

In an example, the electrodes 41A, 42A, 43A, 44A, 45A, and 46A are arranged in parallel to the navel plane XN and on the same plane above the navel plane XN. The electrodes 41B, 42B, 43B, 44B, 45B, and 46B are arranged in parallel to the navel plane XN and on the same plane below the navel plane XN.

The voltage-measuring electrode pairs 41 to 46 (electrodes 41A, 41B, 42A, 42B, 43A, 43B, 44A, 44B, 45A, 45B, 46A, and 46B) are arranged between the vertical position LA and the vertical position LB, that is, in the inter-electrode range RX. The intermediate position of each of the voltage-measuring electrode pairs 41 to 46 coincides with the intermediate position of the current-applying electrode pair 31. In the illustrated example, their intermediate positions coincide with the navel plane XN. The electrode pairs 41 to 46 are arranged on the projected line CS on their positions.

When the measurement of the visceral fat 82 is started, the controlling unit 50 supplies current between the current-applying electrode pair 31. First, the voltage value is detected by the voltage-measuring electrode pair 41. Next, the voltage value is detected by the voltage-measuring electrode pair 42. Subsequently, the voltage values are sequentially detected similarly by the voltage-measuring electrode pairs 43 to 46.

The voltage value of the electrode pair 41 reflects the visceral fat 82 in the vicinity of the right flank 71C. The voltage value of the electrode pair 42 reflects the visceral fat 82 in the vicinity of a part of the right half of the body that is close to the navel 72 as compared with the electrode pair 41. The voltage value of the electrode pair 43 reflects the visceral fat 82 in the vicinity of a part of the right half of the body that is close to the navel 72. The voltage value of the electrode pair 44 reflects the visceral fat 82 in the vicinity of a part of the left half of the body that is close to the navel 72. The voltage value of the electrode pair 45 reflects the visceral fat 82 in the vicinity of a part of the left half of the body that is close to the left flank 71D as compared with the electrode pair 44. The voltage value of the electrode pair 46 reflects the visceral fat 82 in the vicinity of the left flank 71D.

The controlling unit 50 calculates the distribution of the visceral fat 82 at each position in the circumferential direction based on the voltage values of these electrode pairs 41 to 46, and displays the calculation results on the display unit 23. The controlling unit 50 may calculate the total amount of the visceral fat in the abdominal part 71 by averaging the amount of the visceral fat calculated from these voltage values, and display this on the display unit 23.

As thus described above, the present embodiment can provide the following effects in addition to the effects of the second embodiment.
(11) The plurality of voltage-measuring electrode pairs 41 to 46 is arranged on the projected line CS at various positions of the measured body 70. Therefore, the voltage measurement can be performed at various positions between the current-applying electrode pair 31. This makes it possible to measure the volume of the visceral fat 82 more accurately.
(12) The current-applying electrode pair 31 is arranged in the vicinity of the right flank 71C, and the voltage-measuring electrode pair 46 is arranged in the vicinity of the left flank 71D. In an example, the current-applying electrode pair 31 and the voltage-measuring electrode pair 46 are arranged to face the measured body 70. Since this makes it possible to measure the voltage including the voltage at the farthest position where current flows in the abdominal part 71, more accurate measurement can be performed.
(14) The plurality of voltage-measuring electrode pairs 41 to 46 is arranged between the current-applying electrode pair 31. Therefore, the combination of the electrodes included in the electrode pairs can be selected and switched in accordance with the purpose of the measurement.

### Sixth embodiment

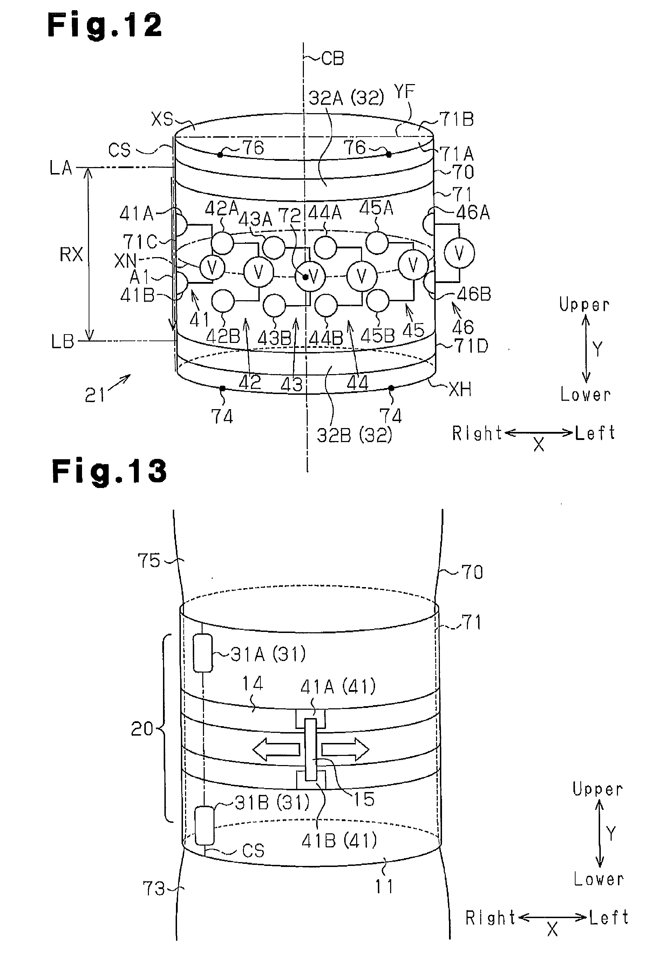

With reference to Fig. 12, a point of the sixth embodiment of the present invention that is different from the fifth embodiment is described. This embodiment is different from the fifth embodiment in the shape and the arrangement of the current-applying electrodes. The other points are similar to those in the fifth embodiment; therefore, the similar structures are denoted by the same reference symbols and description thereof is omitted.

As illustrated in Fig. 12, the detection plane 21 is provided with the current-applying electrodes 32A and 32B, which constitute the current-applying electrode pair 32, and the six voltage-measuring electrode pairs 41 to 46.

Each of the current-applying electrodes 32A and 32B is a band-like electrode. Each of the current-applying electrodes 32A and 32B can have a length corresponding to the distance from the right flank 71C to the left flank 71D of the measured body 70. The current-applying electrodes 32A and 32B are arranged on the detection plane 21 so as to be parallel to the navel plane XN.

As thus described above, the present embodiment can provide the following effects in addition to the effects of the fifth embodiment.
(15) In the present embodiment, since the current-applying electrode 32A is a band-like electrode, current can be applied in a wide range of the front part 71A. The plurality of voltage-measuring electrode pairs 41 to 46 measure the voltage values at their positions, the volume of the visceral fat 82 can be measured accurately.

### Seventh embodiment

With reference to Fig. 13, a point of the seventh embodiment of the present invention that is different from the first embodiment is described. This embodiment is different from the first embodiment in the measuring part 20. The other points are similar to those in the first embodiment; therefore, the similar structures are denoted by the same reference symbols and description thereof is omitted.

As illustrated in Fig. 13, the voltage-measuring electrodes 41A and 41B are movable in the horizontal direction X. For example, the belt 11 can include a movable part 14 for moving the voltage-measuring electrodes 41A and 41B in the horizontal direction X. When the visceral fat measurement device 10 is attached to the measured body 70, the movable part 14 can move in the circumferential direction of the measured body 70. The movable part 14 includes a rail for sliding the voltage-measuring electrodes 41 A and 41B. The voltage-measuring electrode 41A and the voltage-measuring electrode 41B are connected with each other via a connection part 15. When the connection part 15 is moved by a measurer, the electrodes 41 A and 41B are moved integrally. The movable range of the electrodes 41 A and 41B is from the right end position to the left end position of the movable part 14 in the horizontal direction X. In the illustrated example, the movable range of the electrodes 41 A and 41B is from the right flank 71C to the left flank 71D of the measured body 70. While the voltage is measured, the electrodes 41A and 41B are fixed so as to be unmovable. The movable part 14 can have a structure, for example, for fixing the electrodes 41 A and 41B at any position on the movable part 14.

As described above in detail, the present embodiment can provide the following effects in addition to effects similar to the above (1) to (14).
(16) The visceral fat measurement device 10 includes the movable part 14 for moving the voltage-measuring electrodes 41A and 41B on the measured body 70; therefore, the voltage can be measured at various positions on the body surface without the provision of plurality of voltage-measuring electrode pairs.
(17) The voltage-measuring electrode 41A and the voltage-measuring electrode 41B are connected with each other via the connection part 15. Therefore, the voltage can be measured at various positions on the body surface while the relative position between the voltage-measuring electrode 41A and the voltage-measuring electrode 41B is maintained constant.

### Eighth embodiment

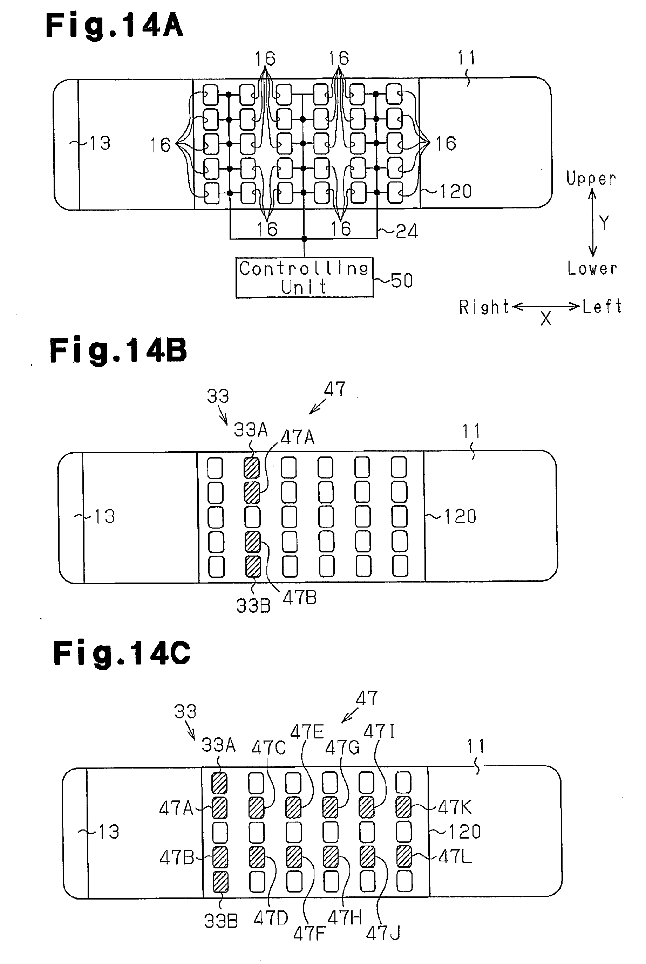

With reference to Figs. 14, a point of the eighth embodiment of the present invention that is different from the first embodiment is described. This embodiment is different from the first embodiment in the measuring part 20. The other points are similar to those in the first embodiment; therefore, the similar structures are denoted by the same reference symbols and description thereof is omitted.

As illustrated in Fig. 14A, a measuring part 120 of the visceral fat measurement device 10 includes a plurality of disposing parts 16 for detachably attaching current-applying electrodes and voltage-measuring electrodes. In the illustrated example, 30 disposing parts 16 are provided in an array form including 5 rows x 6 columns.

The transmission lines 24 connected to the controlling unit 50 are connected to the disposing parts 16. By fitting the current-applying electrodes or the voltage-measuring electrodes in the disposing parts 16, the current-applying electrodes or the voltage-measuring electrodes are electrically connected to the transmission lines 24. Some disposing examples of the current-applying electrodes or the voltage-measuring electrodes are described below.

In the example of Fig. 14B, current-applying electrodes 33A and 33B and voltage-measuring electrodes 47A and 47B are arranged in the second column from the right. The current-applying electrode 33A is attached to the disposing part 16 at the upper end. The current-applying electrode 33B is attached to the disposing part 16 at the lower end. The voltage-measuring electrode 47A is attached to the disposing part 16 below the electrode 33A. The voltage-measuring electrode 47B is attached to the disposing part 16 above the electrode 33B. The current-applying electrodes 33A and 33B constitute a current-applying electrode pair 33, and the voltage-measuring electrodes 47A and 47B constitute a voltage-measuring electrode pair 47. The electrode arrangement of Fig. 14B corresponds to the electrode arrangement of the first embodiment (Fig. 6).

In the example of Fig. 14C, the current-applying electrode 33A is attached to the upper right disposing part 16 and the current-applying electrode 33B is attached to the lower right disposing part 16. Voltage-measuring electrodes 47A, 47B, 47C, 47D, 47E, 47F, 47G, 47H, 47I, 47J, 47K, and 47L are attached within the inter-electrode range RX, that is, to the disposing parts 16 inside the current-applying electrodes 33A and 33B. The intermediate position of the current-applying electrodes 33A and 33B coincides with the intermediate position of the voltage-measuring electrode pair arranged in each column. The current-applying electrodes 33A and 33B constitute the current-applying electrode pair 33, and the voltage-measuring electrodes arranged in each column constitute the voltage-measuring electrode pair. The electrode arrangement of Fig. 14C corresponds to the electrode arrangement of the fifth embodiment (Fig. 11).

As described so far, the present embodiment can provide the following effects in addition to the effects similar to the above (1) to (14).
(18) The visceral fat measurement device 10 includes the disposing parts 16 for detachably attaching the current-applying electrodes and the voltage-measuring electrodes. The belt 11 supports the current-applying electrodes and the voltage-measuring electrodes attached to the disposing parts 16. Since the number and arrangement of the electrodes can be selected, the degree of freedom of measurement for the visceral fat can be increased.

The embodiments above can be modified as below, for example. The modified examples can be combined with each other.

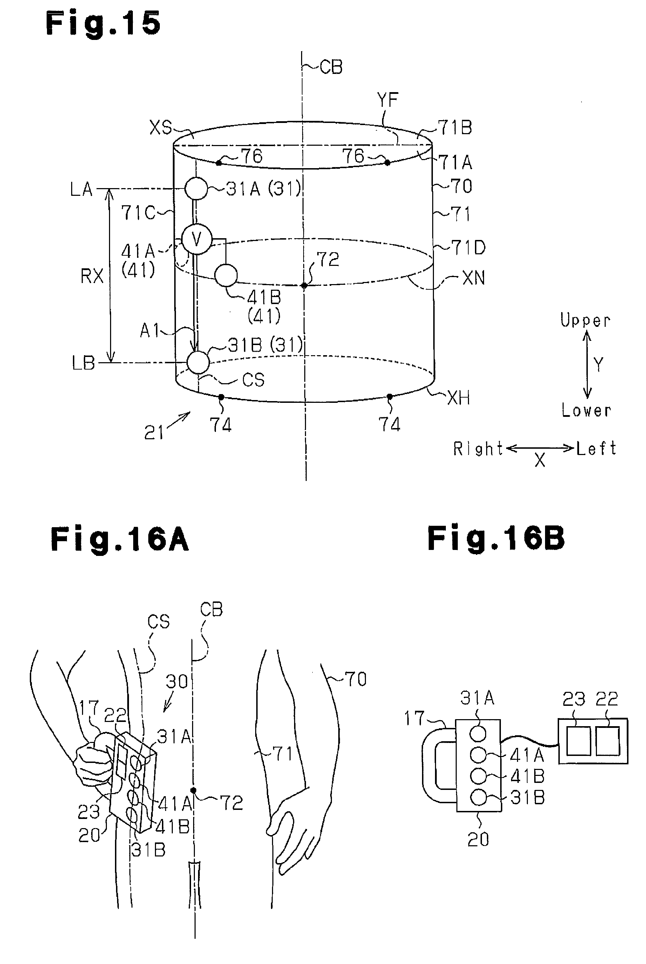

Although the first embodiment provides the electrodes 41A and 41B in the voltage-measuring electrode pair 41 on the inter-electrode line A1 and on the same projected line CS, the arrangement of the voltage-measuring electrode pair 41 is not limited thereto. For example, the electrode 41A and the electrode 41B may not be provided on the inter-electrode line A1, and the voltage-measuring electrode pair 41 may be alternatively provided across the inter-electrode line A1. For example, as illustrated in Fig. 15, the electrode 41A and the electrode 41B may be arranged in parallel to the plane orthogonal to the body trunk axis CB. In this case, the electrode 41A and the electrode 41B are arranged on the same position in the vertical direction Y. Alternatively, the electrode 41A and the electrode 41B may be arranged in parallel to a plane crossing obliquely the body trunk axis CB. In this case, the electrode 41 A and the electrode 41 B are arranged at the different positions from each other in the vertical direction Y.

Although the adjacent electrodes 31A, 41A, 41B, and 31B are arranged at equal intervals in the first embodiment, the intervals may be various. The intervals between the electrodes may be changed as below.

The interval between the electrodes 31A and 31B is made smaller than that in the first embodiment.

The interval between the electrodes 31A and 31B is made larger than that in the first embodiment.

The interval between the electrodes 41A and 41B is made smaller than that in the first embodiment.

The interval between the electrodes 41 A and 41 B is made larger than that in the first embodiment.

In the first embodiment, the voltage-measuring electrode pair 41 arranged on the right half of the body measures the visceral fat 82 in the right half of the body. However, based on the measurement results on the half body by one electrode pair arranged on the half body, the visceral fat 82 of the other half of the body can be estimated. Alternatively, based on the measurement results on the half body, the entire visceral fat amount can be estimated.

In the fifth and sixth embodiments, the voltage-measuring electrode pairs 41 to 46 are arranged in the horizontal direction X. For example, the adjacent electrode pairs 41 to 46 may be arranged at constant intervals or various intervals in the horizontal direction X. The intermediate position of at least one electrode pair does not have to coincide with the navel plane XN.

In the fifth and sixth embodiments, the six current-applying electrode pairs 41 to 46 are provided. However, the number of the voltage-measuring electrode pairs may be seven or more. Alternatively, one to five electrode pairs out of the six electrode pairs 41 to 46 may be omitted.

In the fifth and sixth embodiments, the controlling unit 50 calculates the total amount of the visceral fat of the abdominal part 71 by averaging the amount of the visceral fat calculated from the voltage values of the voltage-measuring electrode pairs 41 to 46. However, the total amount of the visceral fat of the abdominal part 71 may be alternatively calculated by adding the amount of the visceral fat calculated from the voltage values.

In the seventh embodiment, the voltage-measuring electrodes 41A and 41B may be individually movable.

In the seventh embodiment, the electrodes 41A and 41B are fixed so as to be unmovable at the time of the voltage measurement; however, the voltage can be measured while the electrodes 41A and 41B are moved on the measured body 70 along a rail.

In the eighth embodiment, the number and arrangement of the disposing parts 16 can be changed as appropriate.

In the eighth embodiment, each disposing part 16 may be, for example, a hook that can be engaged with the measuring part 120 and the electrode.

In the first, second, fourth, fifth, and seventh embodiments, the current-applying electrode pair 31 may be arranged on the left half of the body.

In the above embodiments, the upper end of the abdominal part 71 is described as the rib plane XS; however, the upper end of the abdominal part 71 may be an upper end of the abdominal cavity, that is, a diaphragm, a breastbone lower end part, or the like. Therefore, the position of the current-applying electrode 31A is not limited to the vicinity of the rib plane XS and below the rib plane XS, and for example, the current-applying electrode 31A may be arranged in the vicinity of the upper end of the abdominal cavity, that is, in the vicinity of the diaphragm or in the vicinity of the breastbone lower end part. The current-applying electrode 31A may be arranged in the vicinity of the rib plane XS and above the rib plane XS as long as the current-applying electrode 31A is in the abdominal part.

In the above embodiments, the lower end of the abdominal part 71 is described as the ilium plane XH; however, the lower end of the abdominal part 71 may be a lower end of the abdominal cavity, a thighbone upper end, or the like. Therefore, the position of the current-applying electrode 31B is not limited to the vicinity of the ilium plane XH and above the ilium plane XH, and the current-applying electrode 31B may be arranged in the vicinity of the lower end of the abdominal cavity or in the vicinity of the thighbone upper end. The current-applying electrode 31B may be arranged in the vicinity of the ilium plane XH and below the ilium plane XH as long as the current-applying electrode 31B is in the abdominal part.

In the above embodiments, the visceral fat measurement device 10 is provided with only one pair of the current-applying electrodes. However, a plurality of current-applying electrode pairs may be provided. As for the measurement method in this case, the controlling unit 50 first applies current to a first current-applying electrode pair and the voltage-measuring electrodes measure the voltage. Next, the controlling unit 50 applies current to a second current-applying electrode pair and the voltage-measuring electrodes measure the voltage. This is repeated for the number of current-applying electrode pairs. Thus, the voltage when current is applied to the different positions is measured at the same position.

In the above embodiments, each electrode is arranged in the front part 71A between the right flank 71C and the left flank 71D; however, the electrode may be arranged in the rear part 71B.

In the above embodiments, the current-applying electrode pair and the voltage-measuring electrode pair are arranged so that their intermediate positions coincide with each other; however, the current-applying electrode pair and the voltage-measuring electrode pair are arranged so that their intermediate positions are different from each other.

In the above embodiments, the visceral fat measurement device 10 includes the belt 11 and the measuring part 20; however, the belt 11 may be omitted. Alternatively, in a visceral fat measurement device 30 illustrated in Fig. 16A, a handle 17 that may be a bar-like shaped and can be held by a measurer is provided instead of the belt 11. The handle 17 may be attached to the measuring part 20. With this structure, a user can measure the visceral fat as having the handle 17 in a state that all the electrodes 31A, 31B, 41A, and 41B are in contact with the body surface and the electrodes 31A, 31B, 41A, and 41B are arranged on the projected line CS. In this case, the measuring part 20 itself functions as the support member. The handle 17 in Fig. 16A may be omitted and the measuring part 20 may be held instead. Alternatively, as illustrated in Fig. 16B, the operating unit 22 and the display unit 23 can be formed separately from the measuring part 20. Note that the handle 17 is not limited to the one illustrated in Figs. 16A and 16B but may be attached to a surface of the measuring part 20 that is opposite to the detection plane 21. Alternatively, the handle may be provided for both side surfaces of the measuring part 20 that face each other. In this case, a user can hold the handles with his/her both hands, so that the user can press the electrodes 31A, 31B, 41A, and 41B against the abdominal part 71.

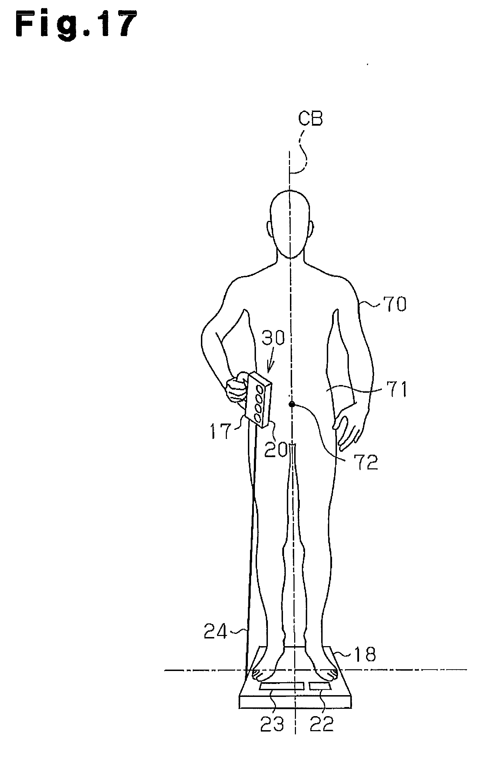

The operating unit 22, the display unit 23, and the controlling unit 50 can be provided at other positions than the belt 11. For example, the visceral fat measurement device 30 illustrated in Fig. 17 includes a separate weighing machine 18 that can communicate with the measuring part 20, and the display unit 23 is provided for the weighing machine 18. With this display unit 23, the measured body 70 can visually recognize the measurement results of the visceral fat 82 easily. One of or both the operating unit 22 and the controlling unit 50 may be provided for the weighing machine 18.

In the above embodiments, the mark 12 is aligned at the rib lower edge 76 and the anterior superior iliac spine 74; however, the mark may be provided at a position corresponding to the navel 72 or the backbone 77. The positions of the electrodes on the visceral fat measurement device 10 may be determined based on this mark.

In the above embodiments, the mark 12 that allows direct positioning with respect to the rib lower edge 76 and the anterior superior iliac spine 74 is provided; however, a scale part protruding upward from the belt 11 may be provided, and the distance from the rib lower edge 76 to the upper end of the belt 11 may be adjusted using this scale part. Alternatively, a scale part protruding upward from the belt 11 may be provided and the distance from the anterior superior iliac spine 74 to the upper end of the belt 11 may be adjusted using this scale part.

In the above embodiments, the measurement results of the visceral fat 82, that is, the volume of the visceral fat 82 is displayed in the three-dimensional image on the display unit 23; however, the measurement results of the visceral fat 82 can be displayed in numerical value. For example, the volume of the visceral fat 82 can be displayed for every part of the measured body 70 like "right part of abdominal part: 50 cm³, upper part of abdominal part: 30 cm³". Moreover, the area of the visceral fat 82 on the navel plane XN (cross section of Fig. 5B) used as a reference of the amount of the visceral fat on the determination of the metabolic syndrome can be displayed.

In the above embodiments, the amount of the visceral fat is calculated based on the measured voltage and this is displayed on the display unit 23; however, the numeral value of the measured voltage can be displayed on the display unit 23.

In the above embodiments, the measurement results of the visceral fat 82 are displayed on the display unit 23; however, the method of transmitting the measurement results to a measurer is not limited thereto. For example, the display unit 23 may be further provided with, or may be replaced by a speaker that transmits the measurement results to the measurer via voice.

In the above embodiments, a conductive gel material may be used as the electrode material.

In the above embodiments, the power supply can be incorporated into the measuring part 20; however, the visceral fat measurement device 10 may be provided with a power supply terminal for receiving power from an external power supply.

The posture of the measured body 70 when the visceral fat is measured is not particularly limited; for example, the measured body 70 may stand up, sit down, or be supine.

In the above embodiments, a human body is described as an example of the measured body 70; however, the measured body 70 may be an animal instead of the human body.
10, 30: visceral fat measurement device; 11: belt; 11A: rear surface; 11B: front surface; 12, 12A, 12B: mark; 13: fastener; 14: movable part; 15: connection part; 16: disposing part; 17: handle; 18: weighing machine; 20, 120: measuring part; 21: detection plane; 22: operating unit; 23: display unit; 24: transmission line; 31 to 39: current-applying electrode pair; 31A, 31B, 32A, 32B, 33A, 33B: current-applying electrode; 41 to 47: voltage-measuring electrode pair; 41A, 41B, 42A, 42B, 43A, 43B, 44A, 44B, 45A, 45B, 46A, 46B, 47A TO 47L: voltage-measuring electrode; 50: controlling unit (computation circuit); 70: measured body; 71: abdominal part; 71A: front part; 71B: rear part; 71C: right flank; 71D: left flank; 72: navel; 73: hip part; 74: anterior superior iliac spine; 75: breast part; 76: rib lower edge; 77: backbone; 80: body fat; 81: subcutaneous fat; 82: visceral fat; 83: muscle.

## Claims

1. A visceral fat measurement device (10) that measures visceral fat by measuring voltage of a measured body when current is applied to the measured body, the visceral fat measurement device (10) comprising:
a measuring part (20); and
a support member (11; 20) having a longitudinal axis,
wherein the measuring part (20) includes
a current-applying electrode pair (31) including a first current-applying electrode (31A) and a second current-applying electrode (31B) for applying current to the measured body, and
a voltage-measuring electrode pair (41) including a first voltage-measuring electrode (41A) and a second voltage-measuring electrode (41B) for measuring voltage of the measured body, wherein
the first current-applying electrode (31A) and the second current-applying electrode (31B) are supported by the support member (11; 20) at an interval on a straight line which is orthogonal to, or substantially parallel to the longitudinal axis of the support member (11; 20), and
the voltage-measuring electrode pair (41) is supported by the support member (11; 20) between the first current-applying electrode (31A) and the second current-applying electrode (31B),
**characterized in that**:
the visceral fat measurement device (10) further includes:
an upper mark (12A) located at an upper end and on a right side from a center of the measuring part (20), and
a lower mark (12B) located at a lower end and on the right side from the center of the measuring part (20), wherein the upper mark (12A) and the lower mark (12B) are configured to serve as positioning marks used for aligning the measuring part (20).

2. The visceral fat measurement device (10) according to claim 1, wherein the support member is configured to support a plurality of voltage-measuring electrode pairs, wherein the voltage-measuring electrode pair (41) is each of, or one of the plurality of voltage-measuring electrode pairs.

3. The visceral fat measurement device (10) according to any one of the preceding claims, comprising a movable part for moving the voltage-measuring electrodes on the measured body.

4. The visceral fat measurement device (10) according to claim 1, comprising a display unit (23) for displaying a visceral fat amount based on a measured voltage of the voltage-measuring electrode pair (41), wherein the display unit (23) is formed separately from the support member (11; 20).

5. The visceral fat measurement device (10) according to claim 1 or 4, wherein the support member (11; 20) includes a belt (11) configured to be wound around the measured body to fix the current-applying electrode pair (31) and the voltage-measuring electrode pair (41) to the measured body.

6. The visceral fat measurement device (10) according to claim 1 or 4, comprising a handle (17) configured to be held by a user at measurement.

7. The visceral fat measurement device (10) according to any one of the preceding claims, wherein the support member (11) includes disposing parts (16) for detachably attaching the current-applying electrodes (31A, 31B) and the voltage-measuring electrodes (41A, 41B).

8. The visceral fat measurement device (10) according to any one of the preceding claims, comprising a computation circuit (50) for calculating a volume of the visceral fat based on a measured voltage of the voltage-measuring electrode pair (41).

9. The visceral fat measurement device (10) according to any one of the preceding claims, comprising a display unit (23) for displaying three-dimensional distribution of the visceral fat based on the measured voltage of the voltage-measuring electrode pair (41).

10. The visceral fat measurement device (10) according to claim 1, wherein
the first current-applying electrode (31A) and the second current-applying electrode (31B) are supported by the support member (11) at an interval on a straight line which is orthogonal to, or substantially parallel to the longitudinal axis of the support member (11), and
the first voltage-measuring electrode (41A) and the second voltage-measuring electrode (41B) are supported by the support member (11) between the first current-applying electrode (31A) and the second current-applying electrode (31B).

11. The visceral fat measurement device (10) according to claim 1, wherein the first current-applying electrode (31A), the second current-applying electrode (31B), the first voltage-measuring electrode (41A), and the second voltage-measuring electrode (41B) are aligned on a common straight line orthogonal to the longitudinal axis of the support member (11; 20).

12. The visceral fat measurement device (10) according to claim 1, wherein
the first current-applying electrode (31A) and the second current-applying electrode (31B) are arranged on a first straight line orthogonal to a longitudinal axis of the support member (11; 20), and
the first voltage-measuring electrode (41A) and the second voltage-measuring electrode (41B) are arranged on a second straight line that is parallel to the first straight line.

13. The visceral fat measurement device (10) according to claim 1, wherein the support member (11) is a flexible support member (11) that can be wound around the measured body.

14. A method for using a visceral fat measurement device (10) according to claim 1, the method comprising:
fixing the support member (11; 20) supporting the current-applying electrode pair (31) and the voltage-measuring electrode pair (41) to a measured body; and
calculating a volume of the visceral fat based on a measured voltage of the voltage-measuring electrode pair (41) when the current-applying electrode pair (31) applies current to the measured body;
**characterized by**
fixing the support member (11; 20) to the measured body while aligning the upper mark (12A) at the rib lower edge (76) of the measured body in the horizontal direction (X) and at the vicinity of the rib plane (XS) and below the rib plane (XS) in the vertical direction (Y) and aligning the lower mark (12B) at the anterior superior iliac spine (74) in the horizontal direction (X) and at the vicinity of the ilium plane (XH) and above the ilium plane (XH) in the vertical direction (Y).

## Patentansprüche

1. Viszeralfett-Messvorrichtung (10), die Viszeralfett durch Messen einer Spannung von einem gemessenen Körper misst, wenn dem gemessenen Körper Strom zugeführt wird, wobei die Viszeralfett-Messvorrichtung (10) umfasst:
ein Messteil (20); und
ein Halteelement (11; 20) mit einer Längsachse,
wobei das Messteil (20) aufweist:
ein Strom zuführendes Elektrodenpaar (31) einschließlich einer ersten Strom zuführenden Elektrode (31A) und einer zweiten Strom zuführenden Elektrode (31 B), um dem gemessenen Körper Strom zuzuführen, und
ein Spannung messendes Elektrodenpaar (41) einschließlich einer ersten Spannung messenden Elektrode (41A) und einer zweiten Spannung messenden Elektrode (41 B), um die Spannung des gemessenen Körpers zu messen, wobei
die erste Strom zuführende Elektrode (31A) und die zweite Strom zuführende Elektrode (31 B) durch das Halteelement (11; 20) in einem Intervall auf einer geraden Linie gehalten werden, die orthogonal oder im Wesentlichen parallel zur Längsachse des Halteelements (11; 20) verläuft, und
das Spannung messende Elektrodenpaar (41) durch das Halteelement (11; 20) zwischen der ersten Strom zuführenden Elektrode (31A) und der zweiten Strom zuführenden Elektrode (31 B) gehalten wird,
**dadurch gekennzeichnet, dass**
die Viszeralfett-Messvorrichtung (10) des Weiteren aufweist:
eine obere Markierung (12A), die sich an einem oberen Ende und an einer rechten Seite von einer Mitte des Messteils (20) befindet, und
eine untere Markierung (12B), die sich an einem unteren Ende und an der rechten Seite von der Mitte des Messteils (20) befindet, wobei
die obere Markierung (12A) und die untere Markierung (12B) so konfiguriert sind, dass sie als Positionierungsmarkierungen dienen, die zum Ausrichten des Messteils (20) verwendet werden.

2. Viszeralfett-Messvorrichtung (10) nach Anspruch 1, wobei das Halteelement konfiguriert ist, um eine Vielzahl von Spannung messenden Elektrodenpaaren zu halten, wobei das Spannung messende Elektrodenpaar (41) jedes oder eines aus der Vielzahl von Spannung messenden Elektrodenpaaren ist.

3. Viszeralfett-Messvorrichtung (10) nach einem der vorhergehenden Ansprüche mit einem beweglichen Teil zum Bewegen der Spannung messenden Elektroden an dem gemessenen Körper.

4. Viszeralfett-Messvorrichtung (10) nach Anspruch 1 mit einer Anzeigeeinheit (23) zum Anzeigen eines Viszeralfettanteils auf der Basis der gemessenen Spannung von dem Spannung messenden Elektrodenpaar (41), wobei die Anzeigeeinheit (23) separat vom Halteelement (11; 20) ausgebildet ist.

5. Viszeralfett-Messvorrichtung (10) nach Anspruch 1 oder 4, wobei das Halteelement (11; 20) einen Gurt (11) aufweist, der so konfiguriert ist, dass er um den gemessenen Körper gewickelt wird, um das Strom zuführende Elektrodenpaar (31) und das Spannung messende Elektrodenpaar (41) an dem gemessenen Körper zu fixieren.

6. Viszeralfett-Messvorrichtung (10) nach Anspruch 1 oder 4 mit einem Handgriff (17), der so konfiguriert ist, dass er beim Messen von einem Anwender gehalten wird.

7. Viszeralfett-Messvorrichtung (10) nach einem der vorhergehenden Ansprüche, wobei das Halteelement (11) Anordnungsteile (16) zum lösbaren Befestigen der Strom zuführenden Elektroden (31A, 31 B) und der Spannung messenden Elektroden (41A, 41B) aufweist.

8. Viszeralfett-Messvorrichtung (10) nach einem der vorhergehenden Ansprüche mit einer Berechnungsschaltung (50) zum Berechnen eines Volumens des Viszeralfetts auf der Basis einer gemessenen Spannung von dem Spannung messenden Elektrodenpaar (41).

9. Viszeralfett-Messvorrichtung (10) nach einem der vorhergehenden Ansprüche mit einer Anzeigeeinheit (23) zum Anzeigen einer dreidimensionalen Verteilung des Viszeralfetts auf der Basis der gemessenen Spannung von dem Spannung messenden Elektrodenpaar (41).

10. Viszeralfett-Messvorrichtung (10) nach Anspruch 1, wobei
die erste Strom zuführende Elektrode (31A) und die zweite Strom zuführende Elektrode (31 B) durch das Halteelement (11) in einem Intervall auf einer geraden Linie gehalten werden, die orthogonal oder im Wesentlichen parallel zur Längsachse des Halteelements (11) verläuft, und
die erste Spannung messende Elektrode (41A) und die zweite Spannung messende Elektrode (41B) durch das Halteelement (11) zwischen der ersten Strom zuführenden Elektrode (31A) und der zweiten Strom zuführenden Elektrode (31 B) gehalten werden.

11. Viszeralfett-Messvorrichtung (10) nach Anspruch 1, wobei die erste Strom zuführende Elektrode (31A), die zweite Strom zuführende Elektrode (31 B), die erste Spannung messende Elektrode (41A) und die zweite Spannung messende Elektrode (41 B) auf einer gemeinsamen geraden Linie orthogonal zur Längsachse des Halteelements (11; 20) ausgerichtet sind.

12. Viszeralfett-Messvorrichtung (10) nach Anspruch 1, wobei
die erste Strom zuführende Elektrode (31A) und die zweite Strom zuführende Elektrode (31 B) auf einer ersten geraden Linie orthogonal zur Längsachse des Halteelements (11; 20) angeordnet sind, und
die erste Spannung messende Elektrode (41A) und die zweite Spannung messende Elektrode (41 B) auf einer zweiten geraden Linie angeordnet sind, die parallel zur ersten geraden Linie verläuft.

13. Viszeralfett-Messvorrichtung (10) nach Anspruch 1, wobei das Halteelement (11; 20) ein flexibles Halteelement (11) ist, das um den gemessenen Körper gewickelt werden kann.

14. Verfahren zur Verwendung einer Viszeralfett-Messvorrichtung (10) nach Anspruch 1, wobei das Verfahren umfasst:
Fixieren des Halteelements (11; 20), das das Strom zuführende Elektrodenpaar (31) und das Spannung messende Elektrodenpaar (41) an einem gemessenen Körper hält; und
Berechnen eines Volumens des Viszeralfetts auf der Basis einer gemessenen Spannung von dem Spannung messenden Elektrodenpaar (41), wenn das Strom zuführende Elektrodenpaar (41) dem gemessenen Körper Strom zuführt;
**gekennzeichnet durch**
Fixieren des Halteelements (11; 20) am gemessenen Körper, während die obere Markierung (12A) am unteren Rippenende (76) des gemessenen Körpers in der horizontalen Richtung (X) und in der Nähe der Rippenebene (XS) und unter der Rippenebene (XS) in der vertikalen Richtung (y) ausgerichtet wird und die untere Markierung (12B) an dem vorderen oberen Darmbeinstachel (Spina iliaca anterior superior) (74) in der horizontalen Richtung (X) und in der Nähe der Darmbeinebene (XH) und über der Darmbeinebene (XH) in der vertikalen Richtung (Y) ausgerichtet wird.

## Revendications

1. Dispositif de mesure de graisses viscérales (10) qui mesure les graisses viscérales en mesurant une tension d'un corps mesuré lorsqu'un courant est appliqué au corps mesuré, le dispositif de mesure de graisses viscérales (10) comprenant:
une partie de mesure (20) ; et
un élément de support (11 ; 20) comportant un axe longitudinal,
où la partie de mesure (20) inclut
une paire d'électrodes d'application de courant (31) incluant une première électrode d'application de courant (31A) et une seconde électrode d'application de courant (31 B) pour appliquer un courant au corps mesuré, et
une paire d'électrodes de mesure de tension (41) incluant une première électrode de mesure de tension (41A) et une seconde électrode de mesure de tension (41B) pour mesurer la tension du corps mesuré, où
la première électrode d'application de courant (31A) et la seconde électrode d'application de courant (31B) sont supportées de manière espacée par l'élément de support (11 ; 20), sur une ligne droite perpendiculaire à, ou substantiellement parallèle à l'axe longitudinal de l'élément de support (11 ; 20), et
la paire d'électrodes de mesure de tension (41) est supportée par l'élément de support (11 ; 20) entre la première électrode d'application de tension (31A) et la seconde électrode d'application de tenson (31B),
**caractérisé en ce que** :
le dispositif de mesure de graisses viscérales (10) inclut en outre :
une marque supérieure (12A) située à une extrémité supérieure et à droite d'un centre de la partie de mesure (20), et
une marque inférieure (12B) située à une extrémité inférieure et à droite d'un centre de la partie de mesure (20), où la marque supérieure (12A) et la marque inférieure (12B) sont conçues pour servir de marques de positionnement utilisées pour aligner la partie de mesure (20).

2. Dispositif de mesure de graisses viscérales (10) selon la revendication 1, où l'élément de support est conçu pour supporter une pluralité de paires d'électrodes de mesure de tension, où la paire d'électrodes de mesure de tension (41) correspond à chacune ou l'une de la pluralité de paires d'électrodes de mesure de tension.

3. Dispositif de mesure de graisses viscérales (10) selon l'une quelconque des revendications précédentes, comportant une partie mobile pour déplacer les électrodes de mesure de tension sur le corps mesuré.

4. Dispositif de mesure de graisses viscérales (10) selon la revendication 1, comportant une unité d'affichage (23) pour afficher une quantité de graisses viscérales sur la base d'une tension mesurée par la paire d'électrodes de mesure de tension (41), où l'unité d'affichage (23) est formée séparément de l'élément de support (11 ; 20).

5. Dispositif de mesure de graisses viscérales (10) selon la revendication 1 ou 4, où l'élément de support (11 ; 20) inclut une ceinture (11) conçue pour être enroulée autour du corps mesuré pour fixer la paire d'électrodes d'application de courant (31) et la paire d'électrodes de mesure de tension (41) sur le corps mesuré.

6. Dispositif de mesure de graisses viscérales (10) selon la revendication 1 ou 4, comportant une poignée (17) conçue pour être tenue par un utilisateur lors de la mesure.

7. Dispositif de mesure de graisses viscérales (10) selon l'une quelconque des revendications précédentes, où l'élément de support (11) inclut des parties d'agencement (16) pour fixer de manière détachable les électrodes d'application de courant (31A, 31B) et les électrodes de mesure de tension (41A, 41B).

8. Dispositif de mesure de graisses viscérales (10) selon l'une quelconque des revendications précédentes, comportant un circuit de calcul (50) pour calculer un volume de la graisse viscérale sur la base d'une tension mesurée par la paire d'électrodes de mesure de tension (41).

9. Dispositif de mesure de graisses viscérales (10) selon l'une quelconque des revendications précédentes, comportant une unité d'affichage (23) pour afficher la distribution tridimensionnelle de la graisse viscérale sur la base de la tension mesurée par la paire d'électrodes de mesure de tension (41).

10. Dispositif de mesure de graisses viscérales (10) selon la revendication 1, où
la première électrode d'application de courant (31A) et la seconde électrode d'application de courant (31B) sont supportées de manière espacée par l'élément de support (11), sur une ligne droite perpendiculaire à, ou substantiellement parallèle à l'axe longitudinal de l'élément de support (11), et
la première électrode de mesure de tension (41A) et la seconde électrode de mesure de tension (41B) sont supportées par l'élément de support (11) entre la première électrode d'application de tension (31A) et la seconde électrode d'application de tension (31B).

11. Dispositif de mesure de graisses viscérales (10) selon la revendication 1, où
la première électrode d'application de courant (31A), la seconde électrode d'application de courant (31B), la première électrode de mesure de tension (41A) et la seconde électrode de mesure de tension (41B) sont alignées sur une ligne droite commune perpendiculaire à l'axe longitudinal de l'élément de support (11 ; 20).

12. Dispositif de mesure de graisses viscérales (10) selon la revendication 1, où
la première électrode d'application de courant (31A) et la seconde électrode d'application de courant (31B) sont agencées sur une première ligne droite perpendiculaire à un axe longitudinal de l'élément de support (11 ; 20), et
la première électrode de mesure de tension (41A) et la seconde électrode de mesure de tension (41B) sont agencées sur une seconde ligne droite parallèle à la première ligne droite.

13. Dispositif de mesure de graisses viscérales (10) selon la revendication 1, où l'élément de support (11) est un élément de support flexible (11) qui peut être enroulé autour du corps mesuré.

14. Procédé pour utiliser un dispositif de mesure de graisses viscérales (10) selon la revendication 1, le procédé comportant les étapes consistant à :
fixer l'élément de support (11 ; 20) supportant la paire d'électrodes d'application de courant (31) et la paire d'électrodes de mesure de tension (41) à un corps mesuré ; et
calculer un volume de la graisse viscérale sur la base d'une tension mesurée par la paire d'électrodes de mesure de tension (41) quand la paire d'électrodes d'application de courant (31) applique un courant au corps mesuré ;
**caractérisée par**
la fixation de l'élément de support (11 ; 20) sur le corps mesuré en alignant la marque supérieure (12A) avec le bord inférieur (76) de la côte du corps mesuré dans la direction horizontale (X) et à proximité du plan de la côte (XS) et sous le plan de la côte (XS) dans la direction verticale (Y) et en alignant la marque inférieure (12B) avec l'épine iliaque antéro-supérieure (74) dans la direction horizontale et à proximité du plan de l'ilium (XH) et au-dessus du plan de l'ilium (XH) dans la direction verticale (Y).
